(19) Europäisches Patentamt
European Patent Office
Office européen des brevets

(11) Publication number: **0 391 433 A2**

(12) # EUROPEAN PATENT APPLICATION

(21) Application number: **90106650.6**

(22) Date of filing: **06.04.90**

(51) Int. Cl.[5]: **G01N 33/86, G01N 33/543, C07K 15/00, C12N 9/74**

(30) Priority: **07.04.89 JP 86785/89**

(43) Date of publication of application: **10.10.90 Bulletin 90/41**

(84) Designated Contracting States: **AT BE CH DE ES FR GB IT LI NL SE**

(71) Applicant: **TEIJIN LIMITED**
**6-7, Minamihonmachi 1-chome Chuo-ku**
**Osaka-shi Osaka 541(JP)**

(72) Inventor: **Koike, Yukiya**
**399-1-102, Miya, Hino-shi**
**Tokyo(JP)**
Inventor: **Murakami, Toshinobu**
**Teijin Musashino-ryo, 3-5-18, Tamadaira**
**Hino-shi, Tokyo(JP)**
Inventor: **Sumi, Yoshihiko**
**4-40-49, Toyoda, Hino-shi**
**Tokyo(JP)**
Inventor: **Ichikawa, Yataro**
**2-11-7, Kotesashi-cho**
**Tokorozawa-shi, Saitama-ken(JP)**

(74) Representative: **Kraus, Walter, Dr. et al**
**Patentanwälte Kraus, Weisert & Partner**
**Thomas-Wimmer-Ring 15**
**D-8000 München 22(DE)**

(54) **Method of immunologically assaying human thrombin-antithrombin III complex, assay reagent and kit therefor.**

(57) A method of immunologically assaying a human thrombin-antithrombin III complex in a human assay sample by using a first antibody composed of an insoluble carrier and an anti-human thrombin polyclonal antibody fixed to the insoluble carrier and a second antibody composed of an anti-human antithrombin III antibody and a labelling substance bound thereto; wherein

(1) the anti-human thrombin polyclonal antibody has

(a) a dissociation constant with respect to human thrombin of $2.5 \times 10^{-9}$M to $3.5 \times 10^{-9}$M,

(b) a dissociation constant with respect to human prothrombin of $4.5 \times 10^{-7}$M to $5.5 \times 10^{-7}$M,

(c) a dissociation constant with respect to the human thrombin-antithrombin III complex of $0.5 \times 10^{-9}$M to $1.5 \times 10^{-9}$M, and

(d) a cross-reactivity with human prothrombin of not more than 0.05 %, and

(2) the anti-human thrombin polyclonal antibody is bound to the insoluble carrier at a rate of 2 to 25 % based on the saturated amount of the polyclonal antibody bound to the insoluble carrier, and a kit therefor.

EP 0 391 433 A2

## BACKGROUND OF THE INVENTION

### 1. Field of the Invention

This invention relates to a method of immunologically assaying human thrombin-antithrombin III complex in a human assay sample, an assay reagent and a kit therefor, a human thrombin polyclonal antibody, a method of producing it, a purified human thrombin-antithrombin III complex, and a method of separating it. More specifically, this invention relates to an immunologically assaying a human thrombin-antithrombin III complex in a human assay sample, particularly a plasma or serum sample, with high sensitivity without serious interference or without influences of prothrombin contained in the human assay sample and of free antithrombin III and free antithrombin III, an assay reagent and kit therefor and to a series of techniques relating to it.

### 2. Description of the Prior Art

Human antithrombin III (to be sometimes called "ATIII" hereinafter) is an important inhibitor of serine protease in the blood coagulation system, and inhibits the activities of thrombin, factors XII, XI, X and IX, kallikrein and plasmin. The reaction of ATIII and serine protease proceeds at a mole ratio of 1:1. The arginine residue of ATIII forms an ester linkage with the serine residue as the center of activity of serine protease. The resulting complex inhibits the activity of serine protease. One such complex is the human thrombin-antithrombin III complex (to be sometimes called "TAT"). The presence and increase of TAT in human blood is considered to show that thrombin is formed by the initiation and activation of the blood coagulation mechanism.

Accordingly, by measuring the amount of TAT in the blood, the behavior of the blood coagulating system would presumably be able to be known. This will make it possible to determine the condition of a patient from the standpoint of blood coagulation, predict in the early stage the progress of the diseased condition to thrombosis formation or disseminated intravascular coagulation (DIC), and to perform a suitable treatment.

Previously, a method using an anti-TAT neoantigen antibody was attempted as an immunological method of measuring TAT from a human assay sample. For example, Herbert L. Lau attempted to measure TAT by an inhibition assay of anti-TAT neoantigen antibody using $^{125}$I-labelled TAT [The Journal of Biological Chemistry, Vol. 255, 5885, Issue of June 25 (1980)].

Pelzer et al. proposed an assay system for TAT in a human assay sample by a sandwich system using an anti-thrombin antibody in a solid-phase antibody and an anti-ATIII antibody as an enzyme-labelled antibody [Thrombosis & Haemostasis, July 14 (1985)].

However, these conventional methods do not necessarily meet the present-day therapeutical needs. For example, the above-described Lau et al. method has difficulty with the selectivity of anti-TAT neoantigen, and moreover, if even a small amount of free thrombin, ATIII or prothrombin is cross-reacting, the measured value is likely to vary drastically because the concentration of TAT is very small as compared with free ATIII or prothrombin. It is only $1/10^5$ in a normal healthy person. The method of Pelzer et al. does not have sufficient sensitivity, and therefore a large amount of a plasma sample must be used. It has the defect that in an assay sample using a plasma system, it undergoes strong serious interference.

## SUMMARY OF THE INVENTION

It is a first object of this invention to provide a method of immunologically assaying TAT in a human assay sample specifically and with high sensi tivity, an assay reagent and a kit therefor.

A second object of this invention is to provide a method of immunologically measuring TAT in a human assay sample which is practical and serves to determine an index of knowing the behavior of the human blood coagulation system, an assay reagent and a kit therefor.

Another object of this invention is to provide an anti-human thrombin polyclonal antibody which can be used in the immunological assay system for TAT in a human assay sample, and a method of producing it.

Still another object of this invention is to provide a very highly pure TAT which can be used as a standard substance in an immunological assay system for TAT in a human assay sample, and a process for obtaining it.

Further objects of this invention will become apparent from the following description.

Investigations of the present inventors have shown that the above objects are achieved by a method of immunologically assaying a human thrombin-antithrombin III complex in a human assay sample using a first antibody having a human thrombin polyclonal antibody fixed to an insoluble carrier and a second antibody having an anti-human antithrombin III antibody bound to a labelling substance; wherein

(1) the anti-human thrombin polyclonal antibody has (a) a dissociation constant with respect to human thrombin of $2.5 \times 10^{-9}$M to $3.5 \times 10^{-9}$M,

(b) a dissociation constant with respect to human prothrombin of $4.5 \times 10^{-7}$M to $5.5 \times 10^{-7}$M,

(c) a dissociation constant with respect to the human thrombin-antithrombin III complex of $0.5 \times 10^{-9}$M to $1.5 \times 10^{-9}$M, and

(d) a cross-reactivity with human prothrombin of not more than 0.05 %, and

(2) the anti-human thrombin polyclonal antibody is bound to the insoluble carrier at a rate of 2 to 25 % based on the saturated amount of the polyclonal antibody bound to the insoluble carrier.

According to this invention, there is further provided (A) an immunological assay reagent and (B) an immunological assay kit.

Immunological assay reagent (A)

It is an assay reagent for immunologically assaying a human thrombin-antithrombin III complex in a human assay sample and is composed of a first antibody comprising an anti-human thrombin polyclonal antibody fixed to an insoluble carrier and a second antibody composed of anti-human antithrombin III antibody and a labelling substance bound to an anti-human antithrombin III antibody; wherein

(1) the anti-human thrombin polyclonal antibody has

(a) a dissociation constant with respect to human thrombin of $2.5 \times 10^{-9}$M to $3.5 \times 10^{-9}$M,

(b) a dissociation constant with respect to human prothrombin of $4.5 \times 10^{-7}$M to $5.5 \times 10^{-7}$M,

(c) a dissociation constant with respect to the human thrombin-antithrombin III complex of $0.5 \times 10^{-9}$M to $1.5 \times 10^{-9}$M, and

(d) a cross-reactivity with human prothrombin of not more than 0.05 %, and

(2) the anti-human thrombin polyclonal antibody is bound to the insoluble carier at a rate of 2 to 25 % based on the saturated amount of the polyclonal antibody bound to the insoluble carrier.

Immunological assay kit (B)

It is a kit for assaying a human thrombin-antithrombin III complex in a human assay sample comprising a combination of

(1) a first antibody composed of an insoluble carrier and an anti-human thrombin polyclonal antibody fixed thereto,

(2) a second antibody composed of an anti-human antithrombin polyclonal antibody and a labelling substance bound thereto,

(3) a dissolving agent,

(4) a washing agent,

(5) a standard substance, and

(6) a substrate for measuring the enzyme activity and a reaction stopper if the labelling substance is an enzyme; wherein

(1) the anti-human thrombin polyclonal antibody has

(a) a dissociation constant with respect to human thrombin of $2.5 \times 10^{-9}$M to $3.5 \times 10^{-9}$M,

(b) a dissociation constant with respect to human prothrombin of $4.5 \times 10^{-7}$M to $5.5 \times 10^{-7}$M,

(c) a dissociation constant with respect to the human thrombin-antithrombin III complex of $0.5 \times 10^{-9}$M to $1.5 \times 10^{-9}$M, and

(d) a cross-reactivity with human prothrombin of not more than 0.05 %, and

(2) the anti-human thrombin polyclonal antibody is bound to the insoluble carrier at a rate of 2 to 25 % based on the saturated amount of the polyclonal antibody on the insoluble carrier.

In the aforesaid method of immunologically assaying TAT in a human assay sample, the reagent and kit therefor, an anti-human thrombin polyclonal antibody specified in (a) to (d) is used, and bound to a solid carrier at a relatively low density described above. This makes it possible to assay TAT in a human assay sample with high activity.

The present invention will be described in detail below.

The anti-human thrombin polyclonal antibody used in the immunological assay system has a selectively strong affinity for human thrombin and TAT, but has weak affinity for human prothrombin and low cross-reactivity.

This means that the anti-human thrombin polyclonal antibody of this invention has the following affinities and cross-reactivity shown in (a) to (d) below.

(a) It has a dissociation constant with respect to human thrombin of 2.5 x $10^{-9}$M to 3.5 x $10^{-9}$M, preferably 3.0 x $10^{-9}$M to 3.4 x $10^{-9}$M.

(b) It has a dissociation constant with respect to human prothrombin of 4.5 x $10^{-7}$M to 5.5 x $10^{-7}$M, preferably 4.8 x $10^{-7}$M to 5.2 x $10^{-7}$M.

(c) It has a dissociation constant with respect to human thrombin-antithrombin III complex of 0.5 x $10^{-9}$M to 1.5 x $10^{-9}$M, preferably 1.0 x $10^{-9}$M to 1.4 x $10^{-9}$M.

(d) It has a cross-reactivity with human prothrombin of not more than 0.05 %, preferably not more than 0.04 %.

The anti-human thrombin polyclonal antibody which meets the above characteristics (a) to (d) can be obtained by strictly specifying an animal to be immunized, the amount of immunization and the number of immunizations as will be described below. The present inventors immunized mice or sheep under various immunizing conditions, but failed to perform immunization or no desired antibody was obtained.

Investigations of the present inventors have shown that with regard to the anti-human thrombin polyclonal antibody used in this invention, the human thrombin itself to be used for immunization is a strong enzyme (protease), and is a very unstable substance which decomposes a protein in a protein present in the blood of an animal to be immunized, or self-digests, and therefore, the anti-human thrombin polyclonal antibody can be obtained by strictly choosing the characteristics of an antigen for immunization, an animal to be immunized, the method of administering the antigen to the animal, the amount of administration, the time interval of immunization and the period of immunization. A method fot producing an anti-human thrombin polyclonal antibody which meets the characteristics (a) to (d) will be described.

(1) Characteristics of an antigen used for immunization

That human thrombin whose active site is not blocked with an inhibitor such as diisopropyl fluorophosphate is used. Specifically, active human thrombin is dissolved in physiological saline in a concentration of 0.2 to 0.5 mg/ml. The human thrombin used here is preferably alpha-thrombin.

(2) Method of administration

A rabbit is immunized with human thrombin. In the first to third times, equal weights of human thrombin and complete Freund's adjuvant are mixed, and the mixture is subcutaneously injected. After the antibody titer is increased to some extent, active human thrombin is dissolved in physiological saline in a concentration of 0.1 to 0.2 mg/ml, and the solution is intravenously injected.

(3) Amount of administration

Considerations are given so that the rabbit is fully stimulated antigenitically. Care must be taken about the amount of administration because as stated above, thrombin is a powerful protease.

Human thrombin is administered to a rabbit in an amount of 0.05 to 0.25 mg/kg, preferably 0.15 to 0.25 mg/kg each time in a total amount immunized of 0.25 to 1.25 mg/kg, preferably 0.5 to 1.0 mg/kg. From the immunized animal, an anti-human thrombin polyclonal antibody is obtained.

(4) Interval and period of immunization

The immunization period is preferably about 30 days. If it is too short, the anti-titer value is not sufficiently increased. If it exceeds 45 days, the amount (proportion) of the antibody bound to native alpha-thrombin markedly decreases. The immunization interval is preferably one week, and the immunization is carried out 1 to 3 times. After the final imunization (intravenous injection), the whole blood is drawn after the lapse of three days.

(5) Purification

The human thrombin used as an antigen is fixed to Sepharose to prepare a column. By using this column, a polyclonal antibody specifically binding human thrombin is affinity-purified from antiserum. After thorough washing, the antibody is diluted with an acetic acid solution. The antibody is dialyzed, concentrated and subjected to antigen fixed ELISA to determine its cross-reactivity with human prothrombin, and the intensity of binding to human thrombin or TAT. It is confirmed that this polyclonal antibody strongly binds to thrombin and TAT and has antibody activity that can be used for immunological assay. It is then used for TAT assay.

The anti-human thrombin polyclonal antibody can of course be used as an intact antibody. It may also be used in the form of its fragment such as Fab, Fab′ or $F(ab')_2$. Preferably it is used as an intact antibody or its $F(ab')_2$ fragment. The intact antibody is most preferred.

The immunological assay system of this invention is characterized in that an anti-human thrombin polyclonal antibody having the characteristics (a) to (d) is bound to an insoluble carrier at a relatively low density. This permits stable assay of TAT in a human assay sample with high sensitivity. The proportion of the anti-human thrombin polyclonal antibody to be bound to the insoluble carrier in this invention is 2 to 25 %, preferably 4 to 20 %, of the amount of saturated binding to the insoluble carrier. If the amount of the antibody bound is less than 2 % of the amount of saturated binding, TAT is difficult to assay with sufficient precision. On the other hand, if its amount is more than 25 % of the amount of saturated binding, serious interference easily occurs, and the assay accuracy tends to be drastically reduced.

The amount of saturated binding is the maximum amount of the anti-human thrombin polyclonal antibody bound to the insoluble carrier under ordinary conditions, and the amount of saturated binding can be measured under the following conditions.

The insoluble carrier is added to a solution of the anti-thrombin polyclonal antibody having a concentration of 100 g/ml (10 mM phosphate buffer containing 0.125 M NaCl, pH 7.4; PBS) so as to infiltrate it. It is allowed to stand for 12 hours at 4 °C. After washing, the amount of the antibody adsorbed on the carrier is defined as the amount of saturated binding.

Examples of the insoluble carrier to which the anti-human thrombin polyclonal antibody of the invention is fixed include polymers such as polystyrene, polyethylene, polypropylene, polyester, polyacrylonitrile, a fluorocarbon resin, crosslinked dextran and polysaccharide, paper, glass, metals, agarose and combinations of these. Of these polystyrene and polypropylene are preferred.

The shape of the insoluble carrier may be, for example, a tray, a sphere, a fiber, a rod, a disc, a receptacle, a cell or a test tube, a tray having wells, a sphere, and a rod are preferred.

The amount of the antibody bound in an amount of 2 to 25 % based on the amount of saturated binding to the insoluble carrier means that in the case of an insoluble carrier having an ordinary smooth surface, about 0.02 to 0.11 g of the polyclonal antibody is bound to each $cm^2$ of the surface of the insoluble carrier.

To bind the polyclonal antibody to the insoluble carrier at a relatively low density in this invention, a relatively simple method of determining the concentration of the antibody is to determine the relation between the concentration and the amount bound of the polyclonal antibody by a simple experiment involving the properties of the insoluble carrier and the concentration of the polyclonal antibody and to determine the concentration of the antibody that will bring the desired amount of binding.

For example, to bind the polyclonal antibody of the invention to a polyethylene well-plate having a smooth surface, an antibody solution having a concentration of 1 μg/ml to 5 μg/ml is prepared, and added to the plate at a rate of 100 μl/well, and after standing overnight at 4 °C, the plate is washed twice with PBS.

A method of measuring the amount of the anti-thrombin polyclonal antibody bound to the insoluble carrier comprises, for example, reacting TAT with a first antibody having a known amount of binding under the same conditions, detecting the anti-thrombin polyclonal antibody by using a labelled anti-ATIII polyclonal antibody, examining the amount of saturated binding to the insoluble carrier by the above method, and comparing the detected amount of the antibody and the saturated amount of binding of the antibody.

There is no particular limitation on the anti-ATIII polyclonal antibody used as the second antibody having a labelled antibody bound thereto. It may be any antibody which binds to ATIII. It may be an intact antibody or an antibody fragment having the essential binding ability, such as a univalent antibody, Fab, Fab′, or $(Fab')_2$. Fab′ is preferred from the standpoint of sensitivity. Such an anti-ATIII polyclonal antibody is obtained by immunizing a rabbit, sheep, etc. with ATIII as an antigen. Fab′ may be obtained by digesting the resulting anti-ATIII polyclonal antibody with pepsin by the method of Nisonoff et al. (see A. Nisonoff et al., Arch. Biochem. Biophys. 89, 230, 1960) or the method of Ishikawa et al. (see Enzyme Immunological Assay Method, 3rd edition, published by Igakushoin), and subjecting the resulting (Fab′)₂ to a reducing reaction.

Binding of the labelling substance to the anti-ATIII polyclonal antibody may be carried out by a conventional method such as the glutaldehyde method or the maleimide method. A method involving bonding the labelling substance to the antibody via its sulfur atom is preferable. In this case, the reactivity of the labelling substance with sulfur atoms may be increased by treating it with maleimide in advance. A labelled anti-ATIII polyclonal antibody is used as a second antibody.

Advantageously, enzymes, fluorescent substances, photogenic substances and radioactive substances may be advantageously used as the labelling substances in this invention. Enzymes are particularly preferred. Examples of the enzymes include peroxidase, alkaline phosphatase, and beta-D-galactosidase. Examples of the fluorescent substances are fluorescein isothiocyanate and phycobiliprotein. Examples of the radioactive substances are $^{125}I$, $^{131}I$, $^{14}C$ and $^{3}H$. Other enzymes which can be used for immunological assay can be used in this invention.

If the labelling substance is an enzyme, a substrate and if required, a color former are used for measuring its activity.

If peroxidase is used as the enzyme, $H_2O_2$ is used as the substrate, and 2,2′-azino-di-[3-ethylbenzothiazolinesulfonic acid]ammonium salt (ABTS), 5-aminosalicyclic acid, O-phenylenediamine, 4-aminoantipyrin, and 3,3′,5,5′-tetramethylbenzene are used as the color former. If alkaline phosphatase is used as the enzyme, O-nitrophenyl phosphate is used as the substrate. If beta-D-galactosidase is used as the enzyme, fluorescein-di-(beta-D-galactopyranoside) or 4-methylumbeliferyl-beta-D-galactopyranoside may be used as the substrate. The human assay sample for assaying TAT immunologically by using the first antibody and the second antibody of the invention may be any human body fluid which contains TAT, for example, blood, urine, or treated or fractionated product thereof. Usually, serum and plasma are suitable.

The human assay sample used in this invention is preferably used after diluting it with a buffer containing 0.6 to 1.6 M of sodium chloride. If the concentration of sodium chloride in the buffer is less than 0.6 M, ATIII is liable to bind directly to the insoluble carrier, and the second antibody undesirably binds to ATIII directly binding to the insoluble carrier. If the concentration of sodium chloride in the buffer is more than 1.5 M, the antigen-antibody reaction is inhibited.

Now, the method of immunologically assaying TAT and the assay reagent and kit for use therein will be described specifically, the measuring method will be described.

## Method of assaying TAT immunologically

The human thrombin polyclonal antibody having the characteristics (a) to (d) is fixed to a suitable carrier such as a plastic receptacle so that its amount bound may be as described above. Then, to avoid non-specific binding between the insoluble carrier and the human assay sample, the surface of the insoluble carrier is coated with a suitable substance such as bovine serum alubumin.

The insoluble carrier to which the first antibody is immobilized is contacted with the assay sample diluted optionally with a buffer at a fixed temperature for a predetermined period of time. During this time, TAT in the human assay sample binds to the first antibody. After washing with a suitable washing solution, a solution (for example, an aqueous solution) of an anti-ATIII polyclonal antibody (second antibody) labelled with a suitable labelling substance (such as an enzyme) is contacted with TAT bound to the first antibody at a fixed temperature for a fixed period of time to perform reaction. The reaction product is washed with a suitable washing solution, and then, the amount of the labelled substance on the second antibody present on the insoluble carrier is measured.

Thus, the amount of TAT in the assay sample can be calculated from the amount of the labelling substance.

## Assay reagent and kit

A reagent for immunologically assaying TAT is composed of the first antibody reagent and the second antibody reagent.

The immunological assay kit for assaying TAT is composed of

(1) the first antibody,

(2) the second antibody,
(3) a dissolving agent,
(4) a washing agent,
(5) a standard substance, and
(6) if the standard substance is an enzyme, a substrate for measuring the activity of the enzyme, and a reaction stopper.

The dissolving agent (3) used in the kit may be any agent normally used for immunological assay, such as an agent containing a phosphate buffer, a Tris-HCl buffer, or an acetete buffer and having a pH of 6.0 to 8.0. The washing agent (4) may be any washing agent which is generally used for immunological assay, such as physiological saline, a phosphate buffer, Tris-HCl buffer, or a mixture thereof. These washing agents may contain a non-ionic surface active agent such as Triton X100, Tween 20 or Brig 35, or an ionic surface active agent such as sodium dodecylsulfate. The standard substance (5) may be purified TAT. In view of the purpose of its use, TAT substantially free from human antithrombin III, its decomposition product or human thrombin may be used. Advantageously, TAT obtained by the method discovered by the present inventors may be used.

## Purified TAT and a process for obtaining it

Investigations of the present inventors have shown that purified TAT can be easily obtained by the following method, and this TAT does not substantially contain human antithrombin III, its decomposition product and human thrombin. Accordingly the resulting purified TAT can be used as a standard substance in the immunological assay system of this invention.

### Process (I)

A process for separating a purified human thrombin-antithrombin III complex from a solution containing this complex, which comprises

(i) contacting a solution containing a human thrombin-antithrombin III complex with an insoluble carrier having heparin immobilized thereto to adsorb the human thrombin-antithrombin III complex on the insoluble carrier,
(ii) washing the insoluble carrier,
(iii) then eluting the human thrombin-antithrombin III complex adsorbed on the insoluble carrier, and
(iv) separating the human thrombin-antithrombin III complex from the resulting eluate.

### Process (II)

A process for producing a purified human thrombin-antithrombin III complex, which comprises

(i) reacting human thrombin with human antithrombin III in a system in which an insoluble carrier having heparin immobilized thereto is present, to thereby form a human thrombin-antithrombin III complex,
(ii) obtaining the human thrombin-antithrombin III complex adsorbed on the insoluble carrier,
(iii) then liberating the human thrombin-antithrombin III complex adsorbed on the insoluble carrier, and
(iv) separating the human thrombin-antithrombin III complex from the resulting solution.

In processes (I) and (II) described above, the insoluble carriers that can be used may be any of those carriers described above for use in the immunological assay.

In process (I), the TAT-containing solution may suitably be a reaction solution obtained by reacting human thrombin and human antithrombin III in a mole ratio of from 1:2 to 2:1. In processes (I) and (II), an aqueous solution of sodium chloride is preferably used as a medium for eluting or liberating TAT from the insoluble carrier having TAT adsorbed thereon. The concentration of this aqueous solution is preferably 0.15 to 0.75 M.

When in step (i) of process (II), TAT is formed in a system in which an insoluble carrier having heparin immobilized thereto is present, human thrombin may be first fed into a receptacle in which the insoluble carrier is present, such as a columnar receptacle filled with the insoluble carrier in the form of beads, and then human antithrombin III may be fed in a reverse order or simultaneously. Preferably, human thrombin is fed first and then the human antithrombin III is fed, and the formed TAT is adsorbed on the insoluble carrier.

By the processes (I) and (II), TAT of high purity substantially free from human antithrombin III, its decomposition product and human thrombin can be obtained, and can be advantageously used as a standard substance in the above immunological assay system.

The following examples illustrate the present invention in detail without any intention of limiting the invention thereby.

## Brief Description of the Drawings

Figure 1 shows the relation between the proportion of an antibody adsorbed on a solid phase and the precision of assay of TAT;

Figure 2 shows the amount of the antibody adsorbed on a solid phase;

Figure 3 shows the relation between the proportion of the antibody adsorbed on a solid phase and the accuracy of assay of TAT;

Figure 4 (1) is an electrophoretic photograph showing the dilution pattern of a reaction product of ATIII and thrombin on a heparin-5PW column (a product of Toso Co., Ltd.), and Figure 4 (2) is an electrophoretic photograph of SDS-PAGE (10-20 %) of purified TAT;

Figure 5 is an elution pattern of TAT formed in a heparin-5PW column (a product of Toso Co., Ltd.);

Figure 6 shows the effect of thrombin on the TAT assay system; and

Figure 7 shows the effect of foreign proteins in plasma on the TAT assay sample.

## EXAMPLE 1

### Production of antithrombin polyclonal antibody

#### a) Immunization of a rabbit with human thrombin

Purified human thrombin was diluted with physiological saline and rabbits were immunized with the diluted solution as follows:-

| Number of days | Amount (mg/kg of body weight) | Remarks |
|---|---|---|
| 1 | 0.2 | Complete Freund's adjuvant, subcutaneous injection |
| 3 | 0.2 | Ditto |
| 10 | 0.2 | Ditto |
| 17 | 0.05 | Physiological saline, intravenous injection |
| 27 | 0.05 | Physiological saline, intravenous injection |
| 30 | - | Whole blood was drawn |

After the whole blood was drawn, it was left to stand overnight at 4 °C in a glass container to obtain antiserum. The amount of the antiserum obtained was 45 to 50 ml per head.

#### b) Purification of an antibody from the rabbit antiserum

The rabbit antiserum (45 ml) obtained in section a) was passed through a Sepharose 4B column, and then passed through a human thrombin-immobilized Sepharose (10 mg thrombin/10 ml Sepharose) at a flow rate of 9 ml/hr. The column was washed with 300 ml of a 10mM phosphate buffer containing 0.5 M NaCl. Then, the antibody adsorbed on the column was eluted with 1 M acetic acid solution (pH 2.5) containing 0.2 M NaCl. The eluted antibody was neutralized with Tris solution and fully dialyzed against physiological saline. The antibody concentration was determined by a protein assay kit (a product of Bio-Rad Co.). Finally, 4.8 mg of antithrombin polyclonal antibody should be purified.

EXAMPLE 2

Measurement of the dissociation constants of anti-thrombin antibody

Purified anti-thrombin antibody was labelled with $^{125}I$ using Immunobeads (a product of Bio-Rad Co.) to prepare an antibody solution.

Purified human thrombin, human prothrombin, human thrombin-antithrombin complex were indidvidually added in a concentration of 1 μg/ml at a rate of 50 μl/well to 96-well microtiter plates (a product of Flow Lab. Co.), and adsorbed overnight at 4 °C. The antigen solution was removed and then 10 mM of phosphate buffer (pH 7.2) containing 1 % BSA and 0.125 M NaCl was added at a rate of 100 μl/well. The plates were allowed to stand at room temperature for 2 hours. After removing the BSA solution, $^{125}I$-labelled anti-thrombin antibody prepared in a concentration of 0.01 to 5 μg/ml was added to the wells of the plates to react them at 37 °C for 2 hours. Then a 10 mM phosphate buffer (pH 7.2) containing 0.05 % Tween 20 was added at a rate of 100 μl/well to wash the wells three times.

The wells were cut out from the plates and put in a plastic test tube, and the $^{125}I$ radioactivity was measured by a gamma counter [the radioactivity bound to an antigen immobilized to a solid phase; bound - (cpm)]. At the same time, the radioactivity of the antithrombin antibody solution before addition to the wells was measured [the total radioactivity of the antibody added: total (cpm)].

The concentration of the anti-thrombin antibody added was plotted on the axis of abscissas, and the ratio of the total-bound [the radioactivity not bound to the antigen immobilized to the solid phase; free - (cpm)] and bound, on the axis of ordinates. From the Scatchard plot, the dissociation constants (KD) were calculated.

Dissociation constants (KD)
Human thrombin: $3.17 \times 10^{-9}$ M
Human prothrombin: $5.0 \times 10^{-7}$ M or more
Human thrombin-antithrombin III complex: $1.26 \times 10^{-9}$ M

EXAMPLE 3

Cross-reactivity with human prothrombin

5 μg/ml of anti-human thrombin antibody was added to a 96-well plate at a rate of 100 μl/well and left to stand overnight at 4 °C to adsorb the antibody on the solid phase of the wells. After removing the antibody solution, PBS containing 1 % BSA was added at a rate of 150 μl/well, and allowed to stand at room temperature for 2 hours to block the solid phase of the wells. Subsequently, $^{125}I$-labelled human thrombin-ATIII complex (TAT) 10 ng/ml (fixed concentration) was mixed with human prothrombin in various concentrations. The mixture was added to the wells at a rate of 100 μl/well, and reacted with the anti-human thrombin antibody on the solid phase at room temperature for 4 hours. The plate was washed three times with PBS containing 0.05 % Tween 20 and 0.1 % BSA. These wells were cut out from the plate and put in a test tube, and the radioactivity (cpm) of each of the wells was measured by a gamma counter.

When human prothrombin in a concentration of at least $5 \times 10^5$ (500 μg/ml) ng/ml was added, the absorbance increased by the influence of prothrombin.

In other words, it was found that even when human prothrombin existed together in a concentration about $5 \times 10^4$ times that of TAT, the anti-human thrombin antibody on the solid phase selectively recognized TAT and bound to it. Thus, the cross-reactivity of the antibody with prothrombin was not more than 0.05 %.

EXAMPLE 4

Determination of the amount of an antibody adsorbed on a solid phase

Polyclonal antibody (0.5 mg) to thrombin was labelled with $^{125}I$ by using the enzymobeads method (Bio-Rad Co.). The proportion of the labelled antibody was 98 %.

The concentration of the labelled antibody was determined to be 0.86 mg/ml by measuring the absorbance at a wavelength of 280 nm. The specific activity was 5.0 x $10^8$ cpm/mg.

The above antibody was diluted with PBS to a concentration of 0.5, 1, 2.5, 5, 10, 20, 40, 80, and 100 µg/ml, and added to 26-well plates (Titertek®, high activated PVC plates) at a rate of 100 µl/well. After standing at 4 °C overnight, the wells were washed twice with PBS.

The wells were cut off from the plates by means of a cutter knife, put in an Eiken tube, and the radioactivity of $^{125}I$ was measured by means of a gamma counter. From the radioactivity values, the amount (µg) of the antibody adsorbed on the solid phase of the wells was calculated.

The saturated amount of the antibody bound to the insoluble carrier (wells) was 0.632 µg. The percentage of the amount of each antibody adsorbed to the insoluble carrier based on the saturated amount of binding is shown in Table 2.

Table 2

| Concentration of the antibody solutin addeds to the wells (µg/ml) | Amount of the antibody adsorbed on the solid phase of the plate (µg) | Percentage based on the saturated amount of binding (%) |
|---|---|---|
| 0.5 | 0.009 | 1.4 |
| 1 | 0.025 | 4.0 |
| 2.5 | 0.062 | 9.8 |
| 5 | 0.124 | 20.0 |
| 10 | 0.248 | 37.4 |
| 20 | 0.459 | 72.9 |
| 40 | 0.608 | 96.5 |
| 80 | 0.632 | 100.3 |
| 100 | 0.630 | 100.0 |

## EXAMPLE 5

Determination of the optimum amount of the antibody adsorbed on the solid phase

An experiment was carried out using 96-well plates to which an unlabelled anti-thrombin antibody was adsorbed. 1 % BSA-PBS was added to the plates at a rate of 150 µl/well to block the solid phase at room temperature for 2 hours. Then, antigen solutions (solution A containing TAT alone in a concentration of 10 ng/ml, and solution B TAT 10 ng/ml + ATIII 50 µg/ml + prothorombin 50 µg/ml) were added to the plates at a rate of 100 µl/well to perform the reaction at room temperature for 1 hour. The wells were washed three times with PBS containing 0.05 % Tween 20 and 0.1 % BSA. Then, a peroxidase-labelled anti-ATIII (Fab′) was added to the wells at a rate of 100 µl/well, and reacted at room temperature for 1 hour. The wells were washed three times, and then a solution of a substrate (ABTS) was added at a rate of 100 µl/well. Thirty minutes later, the absorbance of the solution at 415 nm was measured.

Let the absorbance of the antigen A solution be a, and the absorbance of the antigen B solution be b, then, the concentration accuracy C of TAT is defined as follows.

$C = \frac{b}{a} \times 100\ (\%)$

The accuracy is higher if C is near to 100. If it is lower than 100, it means that the measurement of TAT is affected by ATIII and prothrombin which are present together.

The results are shown in Figure 1.

If the amount of the antibody adsorbed on the solid phase is in the range of 4.0 % to 20 %, TAT can be measured with good accuracy. If this amount of adsorption is too small, no assay sensitivity is obtained. If it is too large, the assay is affected by ATIII and prothrombin.

## EXAMPLE 6

Adsorption of an antibody to solid phases (insoluble carriers) having different surface conditions

Beads having no polished surface (referred to as rough-surface beads A, Ø 6.28 mm, with an unknown surface area) and beads with a fully polished surface (mirror-surface beads B, Ø 6.28 mm, surface area 1.24 $cm^2$) were individually put in a solution of $^{125}I$-labelled anti-human thrombin antibody (0.5 to 80 µg/ml), and left to stand overnight at 4 °C to adsorb the antibody on the solid phase of beads. The beads were washed twice with PBS. Then the radioactivity (cpm) of the antibody adsorbed on the beads was measured. The amount (µg) of the antibody adsorbed on the solid phase of the beads was calculated from the activity (cpm) and the specific activity (cpm/ g) of the antibody solution. It was found that the amount of the antibody adsorbed on the rough-surface beads A was 1.60 times that on the mirror-surface beads B. The surface area of the mirror-surface beads B was 1.24 $cm^2$, and the surface area of the rough-surface beads A was not known because of the raised and depressed areas on the surface. From the ratio of the amounts of the antibodies adsorbed, the surface area of the rough-surface beads B which would be apparently effective for adsorption of the antibody could be calculated as 1.60 times that of the surface area of the mirror-surface beads B, namely 1.24 $cm^2$ x 1.60 = 1.98 $cm^2$.

The saturated amount of the antibody adsorbed on the solid phase (insoluble carrier) is 0.633 µg for the mirror-surface beads B and 1.010 µg for the rough-surface beads A as shown in Figure 2 when the concentrations of these amounts were taken as 100 %. Thus, proportion of each antibody absorptions was determined. As in Example 5, the measuring accuracy of TAT was calculated. The results are shown in Figure 3. It was found that the proportion of adsorption was 4.0 to 20 % both in the rough-surface beads A and the mirror-surface beads B, and TAT could be detected with good accuracy.

## EXAMPLE 7

Method of evaluating a solid phase on which the antibody was adsorbed

Antibody solutions having a concentration of 1 µg/ml and 5 µg/ml were added to the wells of a 96-well plate, and as shown in Example 4, the antibody was adsorbed on the plate. The amount of saturated binding of the antibody on the solid phase of the plate was 0.630 µg. A plate on which an anti-thrombin antibody was adsorbed (the amount of its adsorption was unknown) was separately prepared. As shown in Example 5, after block ing the plate with BSA, 10 ng/ml of TAT was reacted, and the reaction product was detected by a peroxidase-labelled anti-ATIII antibody (Fab′). A substrate (ABTS) was added to perform coloration, and 30 minutes later, the absorbance ($A_{405}$) was measured, and shown in Table 3.

Table 3

| Concentration of the antibody solution added to the wells (μg/ml) | Amount of adsorption to the solid phase of the plate (μg) | Proportion of the antibody based on the saturated amount of binding (%) | Absorbance ($A_{405}$) |
|---|---|---|---|
| 1 | 0.025 | 4.0 | 0.286 |
| 5 | 0.124 | 19.7 | 0.494 |
| X | Y | Z | 0.583 |

It is considered that when the antibody solution has an adsorbance in the range of 0.286 to 0.494, the amount of antibody adsorbed is 4.0 to 19.7 % based on the amount of saturation of binding. The plate having an unknown amount of adsorption had an absorbance of 0.583 which fell outside the range of 0.286 to 0.494. Thus the amount of the antibody adsorbed on the solid phase of this plate was different.

## EXAMPLE 8

### Production of a TAT standard substance (1)

A human ATIII solution (0.5 mg/ml, 1 ml, 20 mM Tris-HCl, 0.15 M NaCl, pH 7.0, solution) was put in a test tube, and with stirring by means of a magnetic stirrer at room temperature, a purified thrombin solution (0.5 mg/ml, 0.3 ml, 20 mM Tris-HCl, 0.15 M NaCl, pH 7.4, solution) was added. At this time, the thrombin solution was added dropwise, and the ATIII solution was added over the course of about 10 minutes. Then, they were reacted for 30 minutes in a constant temperature vessel at 37 °C to form TAT. Finally 40 units/ml of aprotinin (trasyrol) was added to stop the reaction, and the test tube was added to ice (0 °C). Subsequently, by high-performance liquid chromatography using a heparin-5PW column (a product of Toso Co., Ltd.), the unreacted thrombin and ATIII were separated from the formed TAT, and the TAT was purified.

The separation conditions were as follows.

1) Flow rate: 0.5 ml/min.

2) NaCl concentration gradient

Solution A: 20 mM Tris-HCl pH 7.4

Solution B: 20 mM Tris-HCl-1.5M NaCl pH 7.4

| Time (min.) | Mixing proportion | |
|---|---|---|
| | Solution A (%) | Solution B (%) |
| 0 | 100 | 0 |
| 5 | 90 | 10 |
| 65 | 50 | 50 |
| 75 | 0 | 100 |
| 85 | 0 | 100 |
| 90 | 100 | 0 |

Figure 4 (1) shows a TAT elution pattern, and Figure 4 (2) shows the results of SDS-PAGE of the purified TAT.

3) Peaks eluted at 38 to 39 minutes were separated and obtained. The resulting TAT solution was sufficiently dialyzed against 10 mM phosphate buffer (pH 7.4) containing 0.15 M NaCl and then concentrated. The concentration of the complex was determined by using a protein assay kit (a product of Bio-Rad Co.). It was stored in a refrigerator at -40 °C.

### Measurement of the purified TAT

#### SDS-polyacrylamide electrophoresis (SDS-PAGE) of the purified TAT:-

The TAT solution eluted and collected from the heparin column was applied to SDS-polyacrylamide gel (gel concentration 10 to 20 %, Daiichi Chemicals Co., Ltd.) in an amount corresponding to 1 μg and 2 μg, and subjected to electrophoresis. After the electrophoresis, the gel was dyed with CBB (Coumassie Brilliant Blue). The dyed protein bands were detected by using a gel scanner (SPV-9000 supplied by Shimazu Co., Ltd.). As a result, only the dyed bands of TAT protein (molecular weight 95 K to 100 K) could be detected.

Production of TAT standard substance (2)

The purified human thrombin solution (0.5 mg/ml, 0.3 ml, 20 mM Tris-HCl, 0.15 M NaCl, pH 7.4, solution) was applied to a heparin-5PW column (a product of Toso Co., Ltd.) to bind thrombin to heparin. Subsequently, a human ATIII solution (0.5 mg/ml, 1.0 ml, 20 mM Tris-HCl, 0.15 M NaCl, pH 7.4, solution) was applied to a heparin-5PW column, and allowed to stand at room temperature to form a antithrombin-III complex in the column. The heparin-5PW was washed with 2u mM Tris-HCl, pH 7.4, and by using a NaCl concentration gradient, TAT was separated and purified.

The separation-elution pattern of TAT from the heparin-5PW column is shown in Figure 5.

EXAMPLE 9

Preparation of peroxidase-labelled anti-ATIII

polyclonal Fab′

A commercial rabbit antiserum (10 ml) to ATIII was passed through a ATIII-bound Sepharose, and 1.5 mg of the anti-ATIII antibody was purified.

The purified anti-ATIII antibody was subjected to enzyme digestion and gel filtration to obtain 0.3 mg of a Fab′ fraction. Then the Fab′ fraction was mixed with 0.3 mg of peroxidase containing SH. By gel filtration, peroxidase-labelled anti-ATIII polyclonal Fab′ was purified. The concentration was determined by using a protein assay kit.

EXAMPLE 10

Measurement of TAT in human plasma

A) Influence of prothrombin on the assay system

An antithrombin polyclonal antibody was added to a 96-well microtiter plate in a concentration of 2 μg/ml to adsorb it on the solid phase. The saturated amount of the antibody adsorbed on the solid phase of the plate surface was 0.630 μg. The proportion of the antibody adsorbed on the solid phase was 7.8 %.

The solid phase was blocked with bovine serum alubumin, TAT was diluted to various concentrations with a prothrombin-containing solution (25 μg/ml and 50 μg/ml) (diluting solution: 20 mM Tris-HCl-1.5 M NaCl, 0.05 % Tween 20, 1 unit/ml heparin; pH 7.4). The diluted TAT solutions were added to the wells and reacted at 37 °C for 1 hour. The wells were washed three times with a washing solution (20 mM Tris-HCl-0.125 M NaCl pH 7.4 containing 1 % BSA and 0.05 % Tween 20). Peroxidase-labelled anti-ATIII Fab′ diluted to 200 ng/ml with the diluting solution was added to the wells and reacted at 37 °C for 1 hour. The wells were washed three times with the washing solution. Then, a substrate solution was added to form a color. The absorbance of each of the wells was measured by an ELISA ANALYZER (ETY-96 made by Toyo Sokki Co., Ltd.). The results are shown in Figure 6. The addition of 25 μg/ml and 50 μg/ml of prothrombin scarcely affected the measurement of the complex.

b) Influence of foreign proteins in the plasma on the assay system

Plasma diluted to various concentrations with the diluting solution was used instead of prothrombin, and TAT was measured. The results are shown in Figure 7. TAT could be detected without influences of foreign proteins in a plasma sample diluted to 1/16 to 1/4 with a buffer containing 1.5 M NaCl.

## Claims

1. A method of immunologically assaying a human thrombin-antithrombin III complex in a human assay sample by using a first antibody composed of an insoluble carrier and an anti-human thrombin polyclonal antibody fixed to the insoluble carrier and a second antibody composed of an anti-human antithrombin III antibody and a labelling substance bound thereto; wherein

(1) the anti-human thrombin polyclonal antibody has

(a) a dissociation constant with respect to human thrombin of 2.5 x $10^{-9}$M to 3.5 x $10^{-9}$M,

(b) a dissociation constant with respect to human prothrombin of 4.5 x $10^{-7}$M to 5.5 x $10^{-7}$M,

(c) a dissociation constant with respect to the human thrombin-antithrombin III complex of 0.5 x $10^{-9}$M to 1.5 x $10^{-9}$M, and

(d) a cross-reactivity with human prothrombin of not more than 0.05 %, and

(2) the anti-human thrombin polyclonal antibody is bound to the insoluble carrier at a rate of 2 to 25 % based on the saturated amount of the polyclonal antibody bound to the insoluble carrier.

2. The method of claim 1 in which the anti-human thrombin polyclonal antibody is bound to the insoluble carrier in an amount of 4 to 20 % based on the saturated amount of binding to the insoluble carrier.

3. The method of claim 1 in which the anti-human antithrombin III antibody is an anti-human antithrombin III polyclonal antibody or an equivalent fragment thereof.

4. The method of claim 1 in which the human assay sample is used after it is diluted with a buffer containing sodium chloride in a concentration of 0.6 to 1.5 moles/liter.

5. The method of claim 1 in which the human assay sample is serum or plasma.

6. An assay reagent for assaying a human thrombin-antithrombin III complex in a human assay sample by using a first antibody composed of an insoluble carrier and an anti-human thrombin polyclonal antibody fixed to the insoluble carrier and a second antibody composed of an anti-human antithrombin III antibody and a labelling substance bound thereto; wherein

(1) the anti-human thrombin polyclonal antibody has

(a) a dissociation constant with respect to human thrombin of 2.5 x $10^{-9}$M to 3.5 x $10^{-9}$M,

(b) a dissociation constant with respect to human prothrombin of 4.5 x $10^{-7}$M to 5.5 x $10^{-7}$M,

(c) a dissociation constant with respect to the human thrombin-antithrombin III complex of 0.5 x $10^{-9}$M to 1.5 x $10^{-9}$M, and

(d) a cross-reactivity with human prothrombin of not more than 0.05 %, and

(2) the anti-human thrombin polyclonal antibody is bound to the insoluble carrier at a rate of 2 to 25 % based on the saturated amount of the polyclonal antibody bound to the insoluble carrier.

7. The method of claim 6 in which the anti-human thrombin polyclonal antibody is bound to the insoluble carrier in an amount of 4 to 20 % based on the saturated amount of binding to the insoluble carrier.

8. The method of claim 6 in which the anti-human antithrombin III antibody is an anti-human antithrombin III polyclonal antibody or an equivalent fragment thereof.

9. A kit for assaying a human thrombin-antithrombin III complex in a human assay sample comprising a combination of

(1) a first antibody composed of an insoluble carrier and an anti-human thrombin polyclonal antibody fixed thereto,

(2) a second antibody composed of an anti-human antithrombin polyclonal antibody and a labelling substance bound thereto,

(3) a dissolving agent,

(4) a washing agent,

(5) a standard substance, and

(6) a substrate for measuring the enzyme activity and a reaction stopper if the labelling substance is an enzyme; wherein

(1) the anti-human thrombin polyclonal antibody has

(a) a dissociation constant with respect to human thrombin of 2.5 x $10^{-9}$M to 3.5 x $10^{-9}$M,

(b) a dissociation constant with respect to human prothrombin of 4.5 x $10^{-7}$M to 5.5 x $10^{-7}$M,

(c) a dissociation constant with respect to the human thrombin-antithrombin III complex of 0.5 x $10^{-9}$M to 1.5 x $10^{-9}$M, and

(d) a cross-reactivity with human prothrombin of not more than 0.05 %, and

(2) the anti-human thrombin polyclonal antibody is bound to the insoluble carrier at a rate of 2 to 25 % based on the saturated amount of the polyclonal antibody bound to the insoluble carrier.

10. The assay kit of claim 9 in which the anti-human thrombin polyclonal antibody is bound to the insoluble carrier in an amount of 4 to 20 % based on the saturated amount of binding to the insoluble carrier.

11. The kit of claim 9 in which the anti-human antithrombin III antibody is an anti-human antithrombin III polyclonal antibody or an equivalent fragment thereof.

12. An anti-human thrombin polyclonal antibody which has

(a) a dissociation constant with respect to human thrombin of 2.5 x $10^{-9}$M to 3.5 x $10^{-9}$M,

(b) a dissociation constant with respect to human prothrombin of 4.5 x $10^{-7}$M to 5.5 x $10^{-7}$M,

(c) a dissociation constant with respect to the human thrombin-antithrombin III complex of 0.5 x $10^{-9}$M to 1.5 x $10^{-9}$M, and

(d) a cross-reactivity with human prothrombin of not more than 0.05 %.

13. The anti-human thrombin polyclonal antibody of claim 12 which is a rabbit polyclonal antibody.

14. A process for producing the anti-human thrombin polyclonal antibody of claim 12, which comprises immunizing a rabbit with human thrombin in an amount of 0.05 to 0.25 mg/kg of body weight for each time with the total amount of immunization being 0.25 to 1.25 mg/kg, and obtaining an anti-human thrombin polyclonal antibody from the immunized animal.

15. A human thrombin-antithrombin III complex substantially free from a decomposition product of human antithrombin III and human thrombin.

16. A method of separating a purified human thrombin-antithrombin III complex from a solution containing human thrombin-antithrombin III complex, which comprises

(i) contacting a solution containing a human thrombin-antithrombin III complex with an insoluble carrier to which heparin is immobilized to adsorb the human thrombin-antithrombin III complex on the insoluble carrier,

(ii) washing the insoluble carrier,

(iii) eluting the human thrombin-antithrombin III complex adsorbed on the insoluble carrier, and

(iv) separating the human thrombin-antithrombin III complex from resulting eluates.

17. The method of claim 16 in which the solution containing human thrombin antithrombin III complex is a solution obtained by reacting human thrombin with human antithrombin III.

18. The method of claim 16 in which the elution is carried out by using an aqueous solution of sodium chloride.

19. A method of producing a purified human thrombin-antithrombin III complex, which comprises

(i) reacting human thrombin with human antithrombin III in a system where an insoluble carrier to which heparin is immobilized is present to form a human thrombin-antithrombin III complex,

(ii) obtaining the human thrombin-antithrombin III complex adsorbed on the insoluble carrier,

(iii) then liberating the human thrombin-antithrombin III complex adsorbed on the insoluble carrier, and

(iv) separating the human thrombin-antithrombin III complex from the resulting solution.

Fig. 1

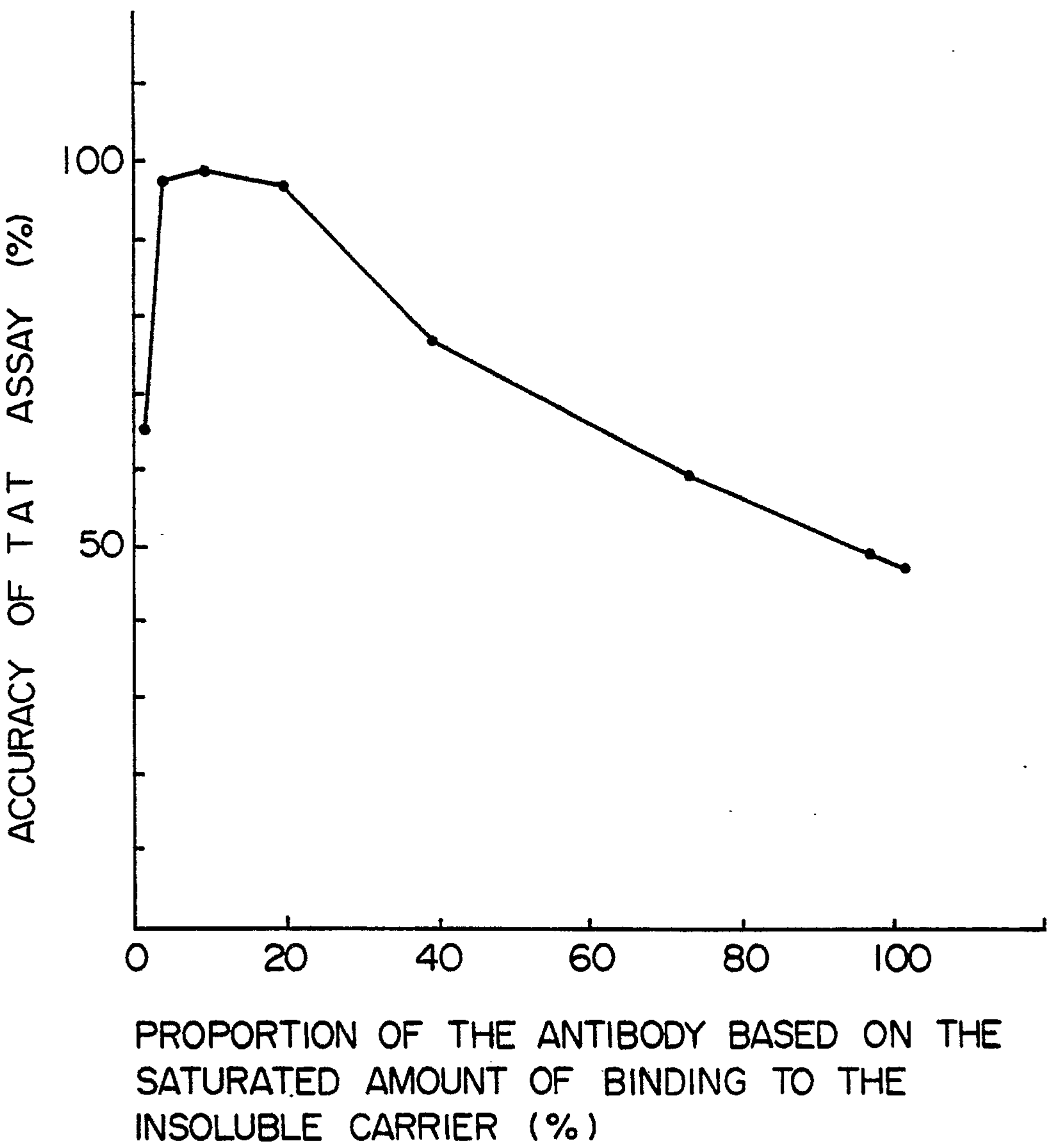
100
50
ACCURACY OF TAT ASSAY (%)
0
20
40
60
80
100
PROPORTION OF THE ANTIBODY BASED ON THE SATURATED AMOUNT OF BINDING TO THE INSOLUBLE CARRIER (%)

Fig. 2

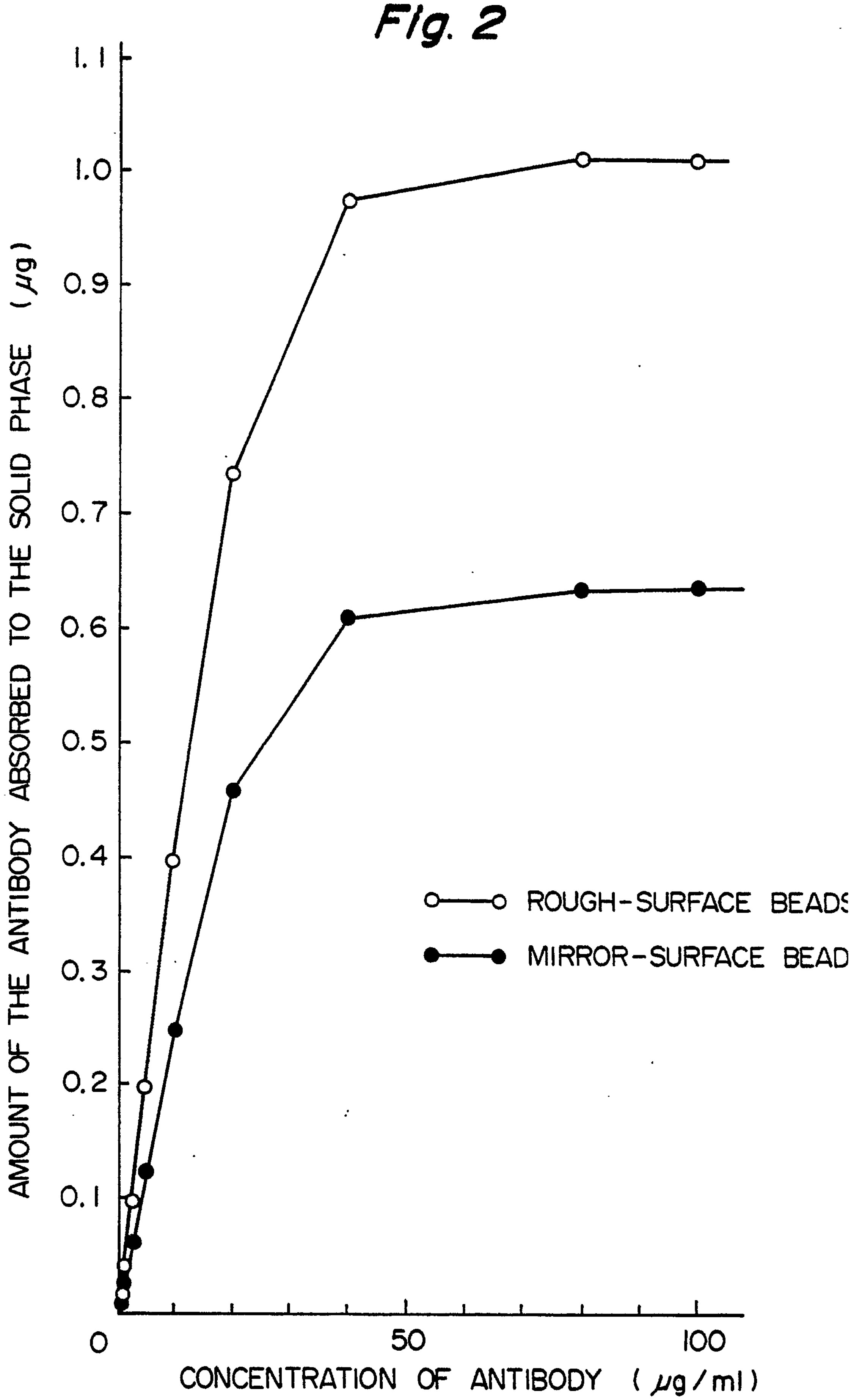
1.1
1.0
0.9
0.8
0.7
0.6
0.5
0.4
0.3
0.2
0.1
AMOUNT OF THE ANTIBODY ABSORBED TO THE SOLID PHASE (μg)
0
50
100
CONCENTRATION OF ANTIBODY (μg/ml)
ROUGH-SURFACE BEADS
MIRROR-SURFACE BEAD

*Fig. 3*

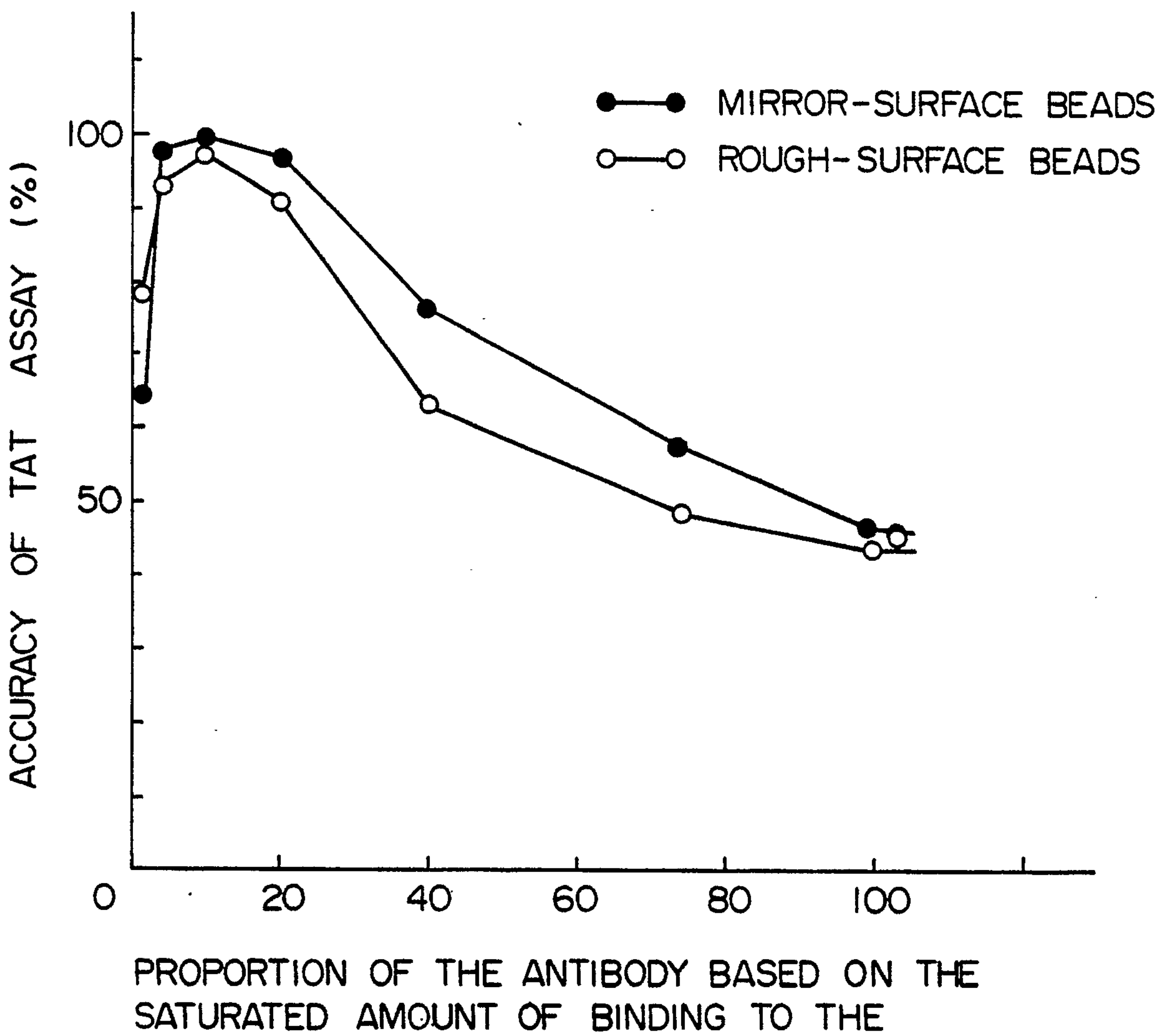
MIRROR-SURFACE BEADS
ROUGH-SURFACE BEADS
ACCURACY OF TAT ASSAY (%)
100
50
0
20
40
60
80
100
PROPORTION OF THE ANTIBODY BASED ON THE SATURATED AMOUNT OF BINDING TO THE INSOLUBLE CARRIER (%)

*Fig. 4(1)*

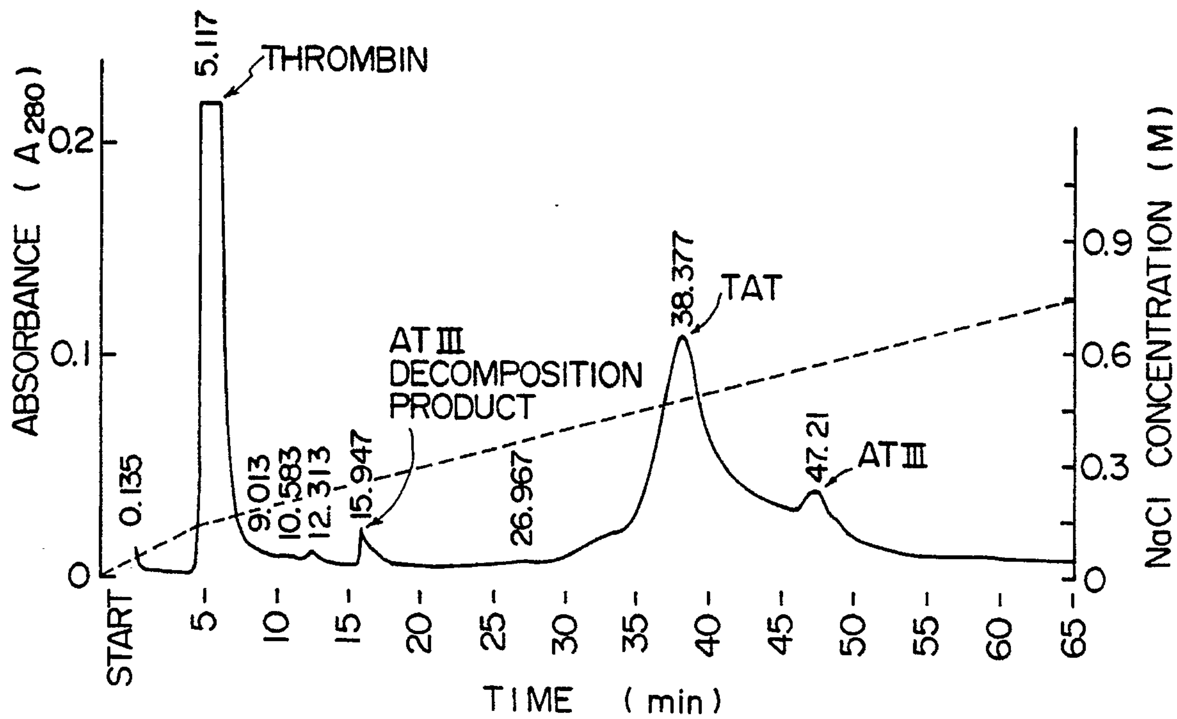
ABSORBANCE ( $A_{280}$ )
0.2
0.1
0
5.117
THROMBIN
0.135
9.013
10.583
12.313
15.947
AT III DECOMPOSITION PRODUCT
26.967
38.377
TAT
47.21
AT III
NaCl CONCENTRATION (M)
0.9
0.6
0.3
0
START
5
10
15
20
25
30
35
40
45
50
55
60
65
TIME (min)

*Fig. 5*

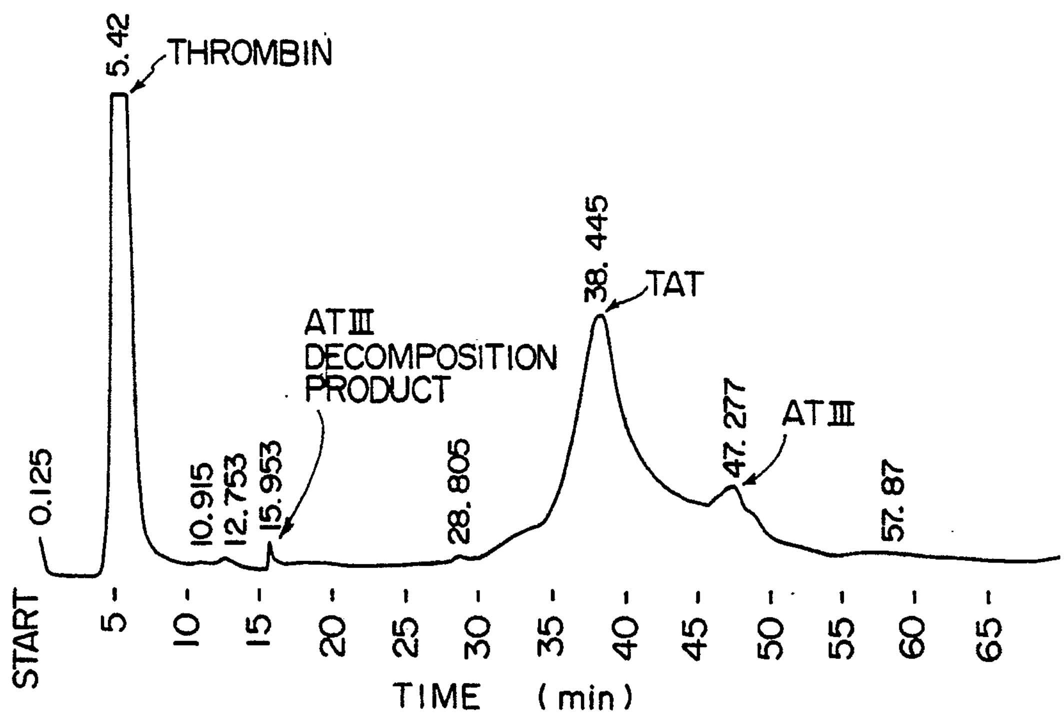
5.42
THROMBIN
0.125
10.915
12.753
15.953
AT III DECOMPOSITION PRODUCT
28.805
38.445
TAT
47.277
AT III
57.87
START
5
10
15
20
25
30
35
40
45
50
55
60
65
TIME (min)

*Fig. 4(2)*

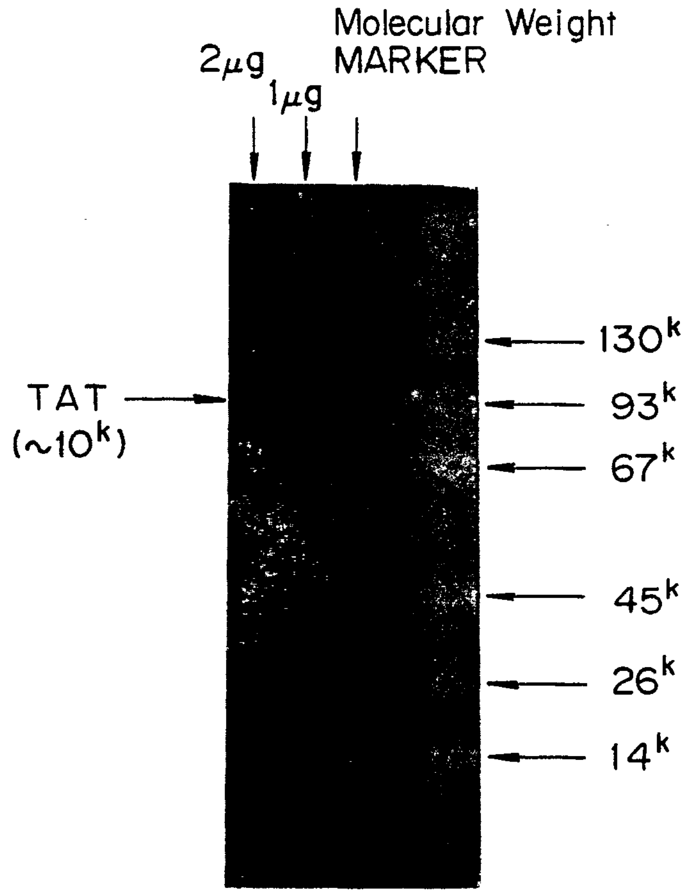

Molecular Weight MARKER
2μg
1μg
TAT
(~10k)
130k
93k
67k
45k
26k
14k

*Fig. 6*

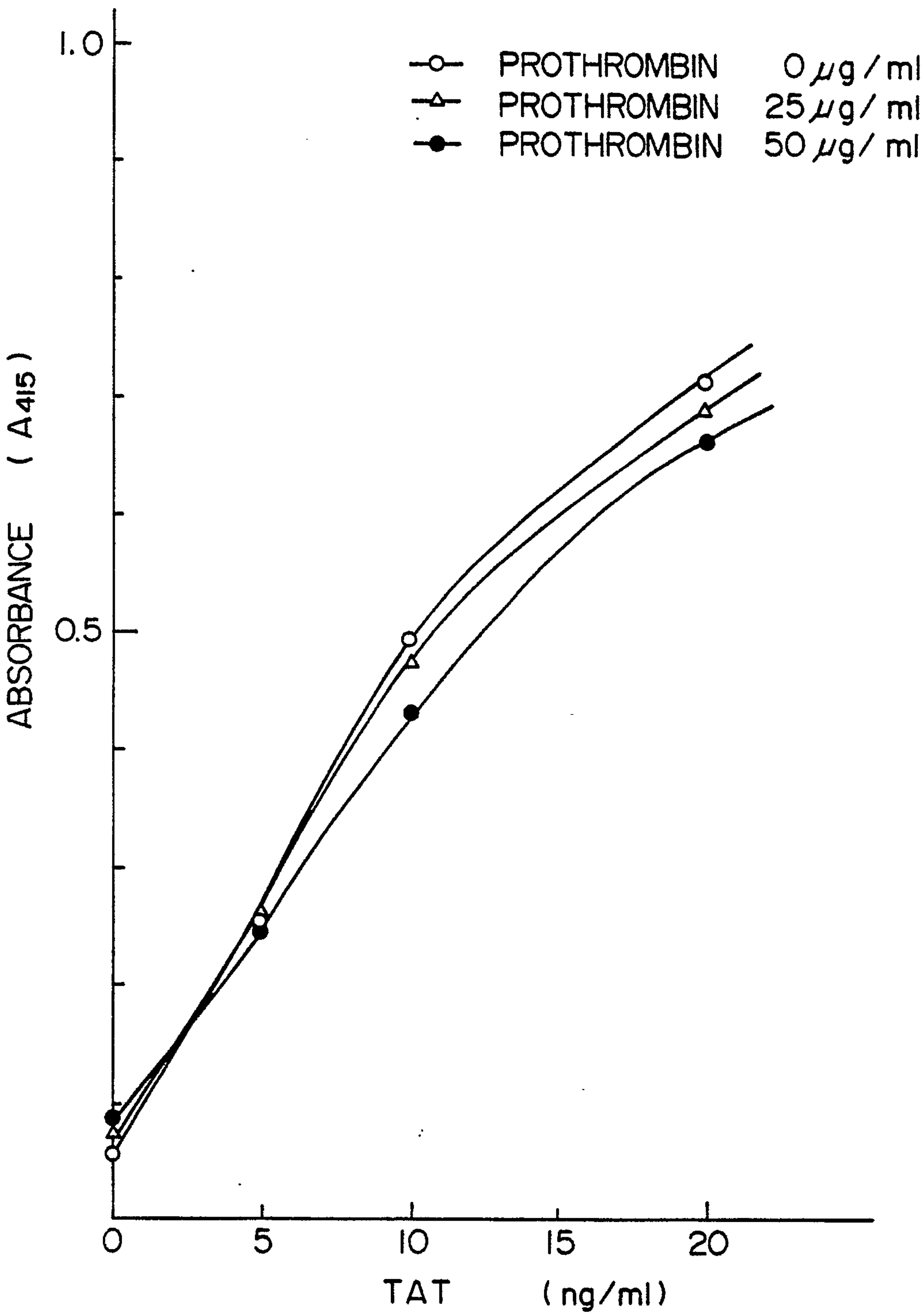
PROTHROMBIN 0 μg/ml
PROTHROMBIN 25 μg/ml
PROTHROMBIN 50 μg/ml
ABSORBANCE (A415)
1.0
0.5
0
5
10
15
20
TAT (ng/ml)

*Fig. 7*

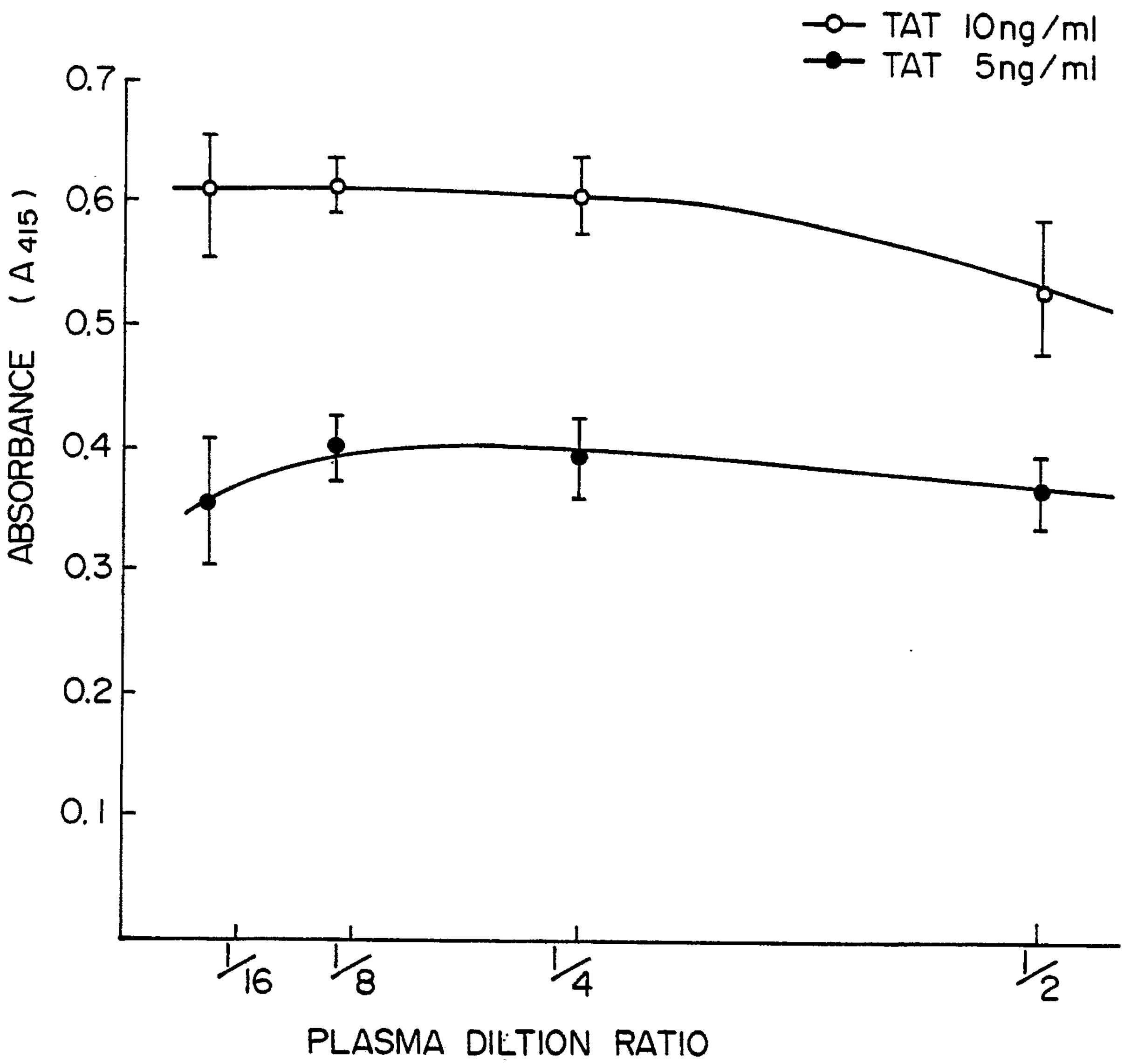
TAT 10ng/ml
TAT 5ng/ml
0.7
0.6
0.5
0.4
0.3
0.2
0.1
ABSORBANCE (A 415)
1/16
1/8
1/4
1/2
PLASMA DILTION RATIO